# EUROPEAN PATENT SPECIFICATION

(11) **EP 4 278 967 B1**
(45) Date of publication and mention of the grant of the patent: **29.01.2025**
(21) Application number: 22174560.7
(22) Date of filing: 20.05.2022
(51) Int. Cl.: A61B 5/145, G01N 21/78, G01N 21/84, G01N 33/52

(54) **METHOD OF DETERMINING A FINAL NUMERICAL ANALYTE RESULT VALUE CORRESPONDING TO A CONCENTRATION OF AN ANALYTE IN A BODILY FLUID USING A MOBILE DEVICE**
VERFAHREN ZUM BESTIMMEN EINES ENDGÜLTIGEN ANALYTE-RESULTATWERTES, DER EINER KONZENTRATION EINES ANALYTS IN EINER KÖRPERFLÜSSIGKEIT ENTSPRICHT, UNTER VERWENDUNG EINER MOBILEN VORRICHTUNG
PROCÉDÉ DE DÉTERMINATION D'UNE VALEUR NUMÉRIQUE FINALE DU RÉSULTAT DE L'ANALYTE CORRESPONDANT À UNE CONCENTRATION D'UN ANALYTE DANS UN FLUIDE CORPOREL À L'AIDE D'UN DISPOSITIF MOBILE

(43) Date of publication of application: 22.11.2023
(73) Proprietor: F. Hoffmann-La Roche AG, 4070 Basel (CH); Roche Diabetes Care GmbH, 68305 Mannheim (DE)
(72) Inventor: BERG, Max, 68305 Mannheim (DE); HAILER, Frederik, 68305 Mannheim (DE); HINZMANN, Rolf, 68305 Mannheim (DE); LIMBURG, Bernhard, 68305 Mannheim (DE)
(74) Representative: Kierig, Elisabeth

(56) References cited:
- EP-A1- 3 825 691
- WO-A1-2020/260246
- WO-A2-2014/025415

## Description

### TECHNICAL FIELD

The present application refers to a method of determining a final numerical analyte result value corresponding to a concentration of an analyte in a bodily fluid using a mobile device having a camera, a processing device, and a display device. The invention further relates to a computer program including computer-executable instructions for performing the method, a non-transitory computer-readable storage medium including instructions that, when executed by a mobile device, cause the mobile device to perform the method, a mobile device having a camera, the mobile device being configured for determining a concentration of an analyte in a bodily fluid, and a kit for determining a concentration of an analyte in a bodily fluid, the kit comprising at least one mobile device and at least one optical test strip having at least one test field.

### BACKGROUND

A number of different devices and methods for determining one or more analytes in body fluids, e.g. blood, urine, interstitial fluid and saliva, are known from prior art. Without narrowing the scope, the invention specifically will be described with respect to blood glucose measurements. It shall be noted, however, that the present invention may also be used for other types of analytical measurements using test elements.

Several test elements are known in the art which comprise at least one test chemical, also referred to as a test reagent, which undergo a coloration reaction in the presence of the at least one Analyte to be detected. Some basic principles on test elements and reagents that may also be used within the scope of the present invention are described e.g. in J. Hönes et al.: Diabetes Technology and Therapeutics, Vol. 10, Supplement 1, 2008, pp.10-26.

In analytical measurements based on color formation reactions, one technical challenge resides in the evaluation of the color change which is due to the detection reaction. Besides using dedicated analytical devices, such as handheld blood glucose meters, the use of generally available electronics such as smart phones and portable computers has become more and more popular over the recent years. Such a method for detecting an analyte concentration using a mobile device is known from EP 3 591 385 A1.

WO 2017/272095 A2 discloses a method comprising the steps of obtaining blood glucose level readings over a diurnal period for each of a plurality of days and of determining an estimated variability of the blood glucose levels over the diurnal period or the plurality of days. The user may adjust blood glucose targets by adjusting their fear of hypoglycemia index (FHI). Said adjustments can add or subtract a predefined value from a previously used blood glucose target.

WO 2017/124006 A1 relates to a method for generating insulin delivery profiles, wherein for a profile blood glucose targets for a particular diurnal period may be set or changed based on historical blood glucose patterns for the patient.

WO 2020/214780 A1 discloses a personalized closed loop system and method for insulin delivery wherein one or more operating mode control parameters are automatically adjusted. Other relevant prior art is disclosed in WO 2020/260246 A1 and WO 2014/025415 A2.

Monitoring analytes relevant for a person's health, it is of particular importance not to miss critical states, i.e. critical analyte concentration, due to the measurement method and/or measurement errors.

It is therefore desirable to provide methods and devices, which address the above mentioned technical challenges of analytical measurements, in particular, when using mobile devices such as consumer-electronics mobile devices, specifically multipurpose mobile devices, which are not dedicated to analytical measurements such as smart phones or tablet computers. Specifically, methods, computer programs, and devices shall be proposed, which are widely applicable to available mobile devices and which are suited to improve reliability and safety while allowing convenient handling for the user.

### BRIEF SUMMARY

This problem is addressed by methods, computer programs, and devices with the features of the independent claims. Advantageous embodiments, which might be realized in an isolated fashion or in any arbitrary combination are listed in the dependent claims.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation, in which one or more further features are present. As an example, the expressions "A has B", "A comprises B" and "A includes B" may all refer to a situation, in which, besides B, no other element is present in A (i.e. a situation in which A solely and exclusively consists of B) and to a situation, in which, besides B, one or more further elements are present in entity A, such as element C, elements C and D, or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect, a method of determining a final numerical analyte result value corresponding to a concentration of an analyte in a bodily fluid using a mobile device having a processing device, and a display device is disclosed. The method includes the steps of determining by the processing device an initial numerical analyte result value from an image of a color formation of a reagent test region, and displaying a final numerical analyte result value and/or an analyte value range of a group of preset analyte value ranges corresponding to the final numerical analyte result value and/or a message corresponding to the corresponding analyte value range. The final numerical analyte result value is determined by the steps of comparing the initial numerical analyte result value to at least one upper threshold value and to at least one lower threshold value and by adding a preset upper bias to the initial numerical analyte result value to form the final numerical analyte result value, if the initial numerical analyte result value exceeds the upper threshold value, or by subtracting a preset lower bias from the initial numerical analyte result value to form the final numerical analyte result value, if the initial numerical analyte result value falls below the lower threshold value, or by using the unchanged initial numerical analyte result value to form the final numerical analyte result value, if the initial numerical analyte result value does neither exceed the upper threshold value nor fall below the lower threshold value.

"Analyte" refers to a substance or chemical constituent that is of interest in an analytical procedure and is also referred to as a component or a chemical species. As an example, one or more analytes may be determined which take part in metabolism, such as blood glucose. Additionally or alternatively, other types of analytes or parameters may be determined, e.g. a pH value. The analyte is comprised in at least one sample of a bodily fluid, and causes a test chemical, also referred to as a test reagent to undergo a color form ation reaction. To cause the reaction, the bodily fluid is applied to a reagent test region, the reagent test region, without limitation, being part of a test strip or the like and comprising the test reagent.

"Bodily fluid" refers to any liquid within the body, such as blood, interstitial fluid, urine, saliva or the like.

"Color formation" refers to a color of the reagent test region resulting from a color formation reaction, i.e., a chemical, biological or physical reaction during which a color, specifically a reflectance, of at least one element involved in the reaction, changes depending on the concentration of the analyte involved in the reaction. The term "color" as used herein is a broad term and may refer to any form of the light reflected of a region comprising the color. In particular, the term "color" also refers to black, white, or grey, as well as to red or green or blue, etc. Thereby, the concentration of the analyte in the bodily fluid may be determined by evaluating the color formation, i.e., the color change due to the reaction, in the reagent test region.

The method is based on using a mobile device for at least a single measurement of an analyte concentration, wherein "mobile device" refers to a mobile electronics device, i.e., a portable device comprising at least one processing device, at least one display device, and, optionally, a camera. The mobile device may specifically refer to a mobile communication device such as a cell phone or a smartphone. Additionally or alternatively, the mobile device may refer to a tablet computer or another type of portable computing device having a display device and, optionally, a camera.

"Processing device" refers to any device capable of processing data such as a data processor and "display device" refers to an output device for presentation of information in visual or tactile form, such as, without limitation, LCD displays, LED displays, or tactile electronics displays.

"Camera" refers to a device having at least one imaging element configured for recording or capturing spatially resolved one-dimensional, two-dimensional or even three-dimensional optical data or information. As an example, the camera may comprise at least one camera chip, such as at least one CCD chip and/or at least one CMOS chip configured for recording images.

Correspondingly, the term "image" relate to data recorded using a camera, such as a plurality of electronic readings from the imaging device, such as the pixels of a chip of the camera. The image may be taken by a camera of the mobile device. Alternatively, the image may be taken by any other camera and transmitted to the processing device of the mobile device by any suitable form of wired or wireless communication.

The camera of the mobile device and/or the camera, the image has been captured with, may be a color camera. Thus, such as for each pixel, color information may be provided or generated, such as color values for three colors R, G, B. A larger number of color values is also feasible, such as four color values for each pixel, for example R, G, G, B. Color cameras are generally known to the skilled person. Thus, as an example, the camera chip may consist of a plurality of three or more different color sensors each, such as color recording pixels like one pixel for red (R), one pixel for green (G) and one pixel for blue (B). For each of the pixels, such as for R, G, B, values may be recorded by the pixels, such as digital values in the range of 0 to 255, depending on the intensity of the respective color. Instead of using color triples such as R, G, B, as an example, quadruples may be used, such as R, G, G, B. The color sensitivities of the pixels may be generated by color filters or by appropriate intrinsic sensitivities of the sensor elements used in the camera pixels. These techniques are generally known to the skilled person.

An initial numerical analyte result value within a sample of the bodily fluid is determined based on an image of a reagent test region. The image may be taken by a camera of the mobile device or by any other camera of the reagent test region after applying the sample of the bodily fluid to the reagent test region. The color formation, i.e. the color of the reagent test region in the image, is determined by the processing device and the initial numerical analyte result value corresponding to the determined color formation is then identified by the processing device, wherein "initial numerical analyte result value" refers to a numerical indication of an analyte concentration in a bodily fluid sample corresponding to the color formation.

To communicate a result to a user a final numerical analyte result value is displayed on a display device of the mobile device. Additionally or alternatively, an analyte value range corresponding to the final numerical analyte result value and/or a message corresponding to the corresponding analyte value range are displayed on a display device of the mobile device. An associated or corresponding "message" refers to any displayable sign capable of informing the user of the status of the analyte concentration range, i.e., of the determined analyte value range. The message may comprise one or more of a range name, corresponding values like the range limit values, a corresponding recommendation, an alarm, a symbol, and the like.

"Analyte value range" refers to a portion of a measurement range, i.e., a range of measurable values, wherein the analyte value range extends between a lower range limit and an upper range limit, i.e., comprises all values of the measurement range between the upper range limit and the lower range limit. Specifically, the measurement range may be portioned by a series of analyte value range in such a way that all values of the measurement range belong to one of the analyte value ranges. For example, the upper range limit of an analyte value range corresponds to the lower range limit of an adjacent analyte value range, wherein the range limit value belongs to one of those adjoining analyte value ranges and the other range comprises all values smaller or larger than that range limit value, respectively. The analyte value range corresponding to a final numerical analyte value is the range comprising the respective final numerical analyte result value or in other words, the final numerical analyte result value falls within the corresponding analyte value range.

Typically for any analyte in a bodily fluid certain concentration values are "normal" while other concentration values are "out of range", either too high or too low and may have negative health impacts. For example, glucose concentration values between 71mg/dl to 130 mg/dl are typically classified as in range, while concentration values below 71 mg/dl are typically classified as a hypoglycemic state and concentrations above 180 mg/dl are typically classified as a hyperglycemic state. The analyte value ranges may represent such preset categories or classes of analyte concentrations.

The final numerical analyte result value is determined based on the initial numerical analyte result value, in particular, the final numerical analyte result value either corresponds to the initial numerical analyte result value or results from biasing the initial numerical analyte result value depending on the result of a comparison of the initial numerical analyte result value with at least two threshold values.

It shall be understood that the biases, i.e. the upper bias and the lower bias, each comprise one or more positive values, so that initial numerical analyte result values exceeding the upper threshold value are increased by adding the upper bias to form the final numerical analyte result value and initial numerical analyte result value falling below the lower threshold value are decreased by subtracting the lower bias to form the final numerical analyte result value.

Furthermore, in some embodiments the method steps are repeated regularly, e.g. on demand by a user, or following a preset schedule, or due to trigger events or messages, or the like.

As with any measurement the numerical analyte result values determined by the method show measurement errors, i.e., a difference between the determined initial numerical analyte result value and the actual analyte concentration value within the bodily fluid. The error distribution may be assumed to have a symmetric, e.g. Gaussian, shape. Therefore, for any actual concentration value of a bodily fluid sample that is very close to or identical to a range limit between two adjacent analyte value ranges the actual analyte concentration has a probability of exactly 50% or about 50% to be associated with either of the two adjacent analyte value ranges.

Regarding the range limits with "extremer" ranges, i.e., analyte value ranges covering small or very small or high or very high measurement values, respectively, this bears the risk of missing out those "extremer" ranges and erroneously communicating a final numerical analyte result value or a corresponding analyte value range that is less extreme. In other words, erroneously missing to attribute an extremer final numerical analyte result value or analyte value range might imply missing warning a user about a critical state with regard to the analyte concentration in his bodily fluid, e.g., a critically high or low blood glucose concentration, which might have health or even life threatening consequences.

With the artificial biasing of all initial numerical analyte result values below or above respective thresholds, the likelihood of higher or lower result values to represent "extremer" states and fall into "extremer" ranges is increased. Therewith the likelihood of missing critically high or low analyte concentrations is decreased, which increases the reliability and safety of the measurement method.

On the other hand, it is much less critical if an actual in-range-concentration is falsely categorized to belong to a more extreme range and a corresponding "extremer" result value or range is communicated to the user. The preferred communication of more critical states may even have a favorable effect on user behavior.

In one embodiment, the upper bias is a constant upper offset value or an upper transition function with function values depending on the initial numerical analyte result value and/or on an absolute value of a difference between the initial numerical analyte result value and the upper threshold value.

In one embodiment, the lower bias is a constant lower offset value or a lower transition function with function values depending on the initial numerical analyte result value and/or on an absolute value of a difference between the initial numerical analyte result value and the lower threshold value.

The term "bias" here is used, without limitation, for a constant positive value or for a function. A constant upper offset value results in increasing all initial numerical analyte result values exceeding the upper threshold value by a constant value. Correspondingly, a constant lower offset value results in decreasing all initial numerical analyte result values falling below the lower threshold value by a constant value.

Alternatively, the upper or lower bias are an upper or lower transition function so that the specific bias value added to or subtracted from an initial numerical analyte result value to form the final numerical analyte result value depends on the distance of said numerical analyte result value to the respective threshold value. "distance" here refers to the absolute value of a difference between the initial numerical analyte result value and the respective threshold value. "Absolute value" as used herein is a broad term and is to be given its ordinary and customary meaning to a person of ordinary skill in the art and is not to be limited to a special or customized meaning. The absolute value of a number, also referred to as modulus, specifically may refer to a non-negative value of said number without regard to its sign.

In one embodiment, the lower offset value and the upper offset value are identical or differ from each other.

With increasing the size of the offset values, the probability of a final numerical analyte result value to fall into an extremer analyte value range is increased. For example the size of the offset values may be chosen by taking into account the size of measurement errors to be expected and this may prompt to select differing offset values for the upper offset value and the lower offset value, respectively. For example, with a greater upper bias, i.e., the upper offset value being greater than the lower offset value, to be added to any initial numerical analyte result value exceeding the upper threshold value, it may be accounted for the relative measurement error being greater for greater values.

In one embodiment, the upper transition function and/or the lower transition function gradually increases with an increase of distance given by the absolute value of the difference between the numerical analyte result value and the upper threshold value or lower threshold value, respectively.

"To gradually increase" or a "gradual increase" refers to continuous or stepwise increase. Thus, here a gradually increasing function refers to a function with function values that with increasing distance from the respective threshold value either increase and/or in sections remain constant such as a linear function with function values being proportional to the distance or an increasing step function or combinations thereof. Therewith, the upper bias and/or the lower bias gradually increases with increasing distance, so that the increase of the initial numerical analyte result value exceeding the upper threshold value and/or the decrease of the initial numerical analyte result value falling below the lower threshold value is increasing gradually.

In one embodiment, the upper transition function and/or the lower transition function includes a first region with a gradual increase with an increase of the respective distance and a second region with a constant value. Here "region" refers to a section or segment of the transition function and may be defined by specifying range of initial analyte result values.

In an embodiment the first region extends between the upper threshold value and a greater second upper threshold value or between the lower threshold value and a smaller second lower threshold value, respectively, while the second region is adjacent to the first region, covering all values greater than the second upper threshold value or smaller than the second lower threshold value, respectively. It shall be understood that each threshold value belongs to one of the two adjacent regions.

In one embodiment, the gradual increase of the upper transition function and/or the lower transition function is proportional to the absolute value of the difference between the numerical analyte result value and the upper threshold value or lower threshold value, respectively.

In one embodiment the proportional correlation applies to the upper or lower transition function as whole. Alternatively, the proportional correlation applies to a region of a measurement range, i.e., to a portion or part of possible initial numerical analyte result value, for example to the first region.

In one embodiment, the upper threshold value and the lower threshold value are identical.

If the upper and lower threshold values differ from each other, the final numerical analyte result values are identical to the initial numerical analyte result values comprised by a central region between the two thresholds, while in the regions above the upper threshold value and below the lower threshold value the final numerical analyte result value are formed by increasing or decreasing the initial numerical analyte result value, respectively.

If the upper and lower threshold values are identical, all or all but the one initial numerical analyte result value being identical to the threshold value, are formed by biasing the initial numerical analyte result value.

In one embodiment, determining the final numerical analyte result value includes comparing the initial numerical analyte result value to a second upper threshold value and a second lower threshold value and using the unchanged initial numerical analyte result value to form the final numerical analyte result value if the initial numerical analyte result value exceeds the second upper threshold value or falls below the second lower threshold value.

The second lower threshold value may be smaller than the lower threshold value and the second upper threshold value greater than the upper threshold value, so that for all initial numerical analyte result value falling below the lower threshold value and exceeding the second lower threshold value, the final numerical analyte result value is formed by subtracting the lower bias from the initial numerical analyte result value, while for all initial numerical analyte result value falling below the second lower threshold value the final numerical analyte result value is formed by the unchanged initial numerical analyte result value. Correspondingly, only for the initial numerical analyte result value being greater than the upper threshold value and smaller than the second upper threshold value the final numerical analyte result value is formed by biasing the initial numerical analyte result value, i.e., by adding the upper bias. Therewith the biasing step may be saved/omitted for "very extreme" values as it is not necessary to ensure not to miss critical analyte concentrations for such final numerical analyte result values, for which the probability to be categorized as critical, is very high, e.g. 70% or 90% or 95% or even higher, the.

It may suffice to bias only those concentration values, i.e. initial numerical analyte result value, that classify as in range but are close or very close to more critical values, e.g. close to a category boarder or range limit of a category/range specified as out-of-range. Alternatively, it may suffice to bias additionally those initial numerical analyte result value that classify as slightly out of range.

In one embodiment, the analyte is blood glucose and the bodily fluid is either blood or interstitial fluid.

In another embodiment, the upper threshold value is between 160 mg/dl - 180 mg/dl or between 165 mg/dl - 175 mg/dl or between 168 mg/dl - 172 mg/dl or between 169 mg/dl - 171 mg/dl. In a further embodiment, the lower threshold value is between 60 mg/dl - 90 mg/dl or between 75 mg/dl - 85 mg/dl or between 78 mg/dl - 82 mg/dl or between 79 mg/dl - 81 mg/dl.

In another aspect, a computer program is disclosed including computer-executable instructions for performing the method described above.

In a further aspect, a non-transitory computer-readable storage medium is disclosed, the computer-readable storage medium including instructions that when executed by a mobile device, cause the mobile device to perform the method as described above.

In another aspect, a mobile device is disclosed including a processing device, a display device, a camera, and a memory storing instructions that, when executed by the processing device, configure the mobile device to perform the method as described above.

In a further aspect, a kit for determining a concentration of an analyte in a bodily fluid, the kit includes at least one mobile device as described above and at least one optical test strip having at least one test field.

Other technical features of the different aspects of the invention may be readily apparent to one skilled in the art from the following figures, descriptions, and claims.

### BRIEF DESCRIPTION OF THE SEVERAL VIEWS OF THE DRAWINGS

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the figures.
FIG. 1 illustrates a flowchart of an embodiment of a method of determining a final numerical analyte result value corresponding to a concentration of a bodily fluid using a mobile device.
FIG. 2 illustrates the artificial biasing of measurement results in accordance with a first embodiment.
FIG. 3 illustrates the artificial biasing of measurement results in accordance with a second embodiment.
FIG. 4 illustrates the artificial biasing of measurement results in accordance with a third embodiment.
FIG. 5 illustrates the artificial biasing of measurement results in accordance with a fourth embodiment.
FIG. 6 illustrates the artificial biasing of measurement results in accordance with a fifth embodiment.
FIG. 7 illustrates the effect of measurement errors regarding missing critically low analyte concentration states.
FIG. 8 illustrates the effect of measurement errors regarding missing critically high analyte concentration states.

### DETAILED DESCRIPTION

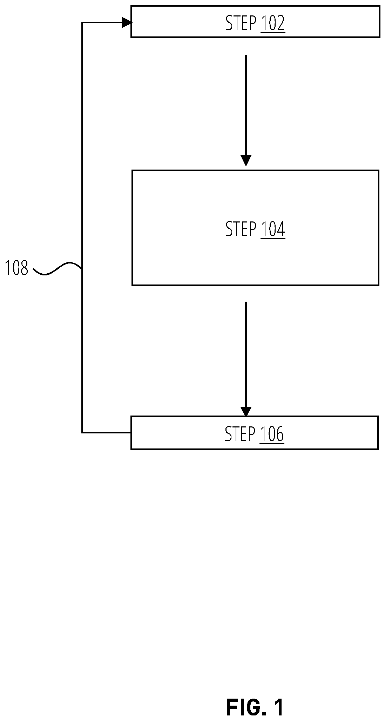

In FIG. 1 a method of determining a final numerical analyte value corresponding to a concentration of an analyte in a bodily fluid is schematically shown. The method is executed by a mobile device comprising a processing device, a display device, and optionally a camera. In a first step 102 an initial numerical analyte result value is determined from a color formation of a reagent test region. The image is taken by the camera of the mobile device or by any other camera and transferred to the mobile device and the determination process is executed by the processing device. The reagent test region may form part of a test strip and may comprise a test reagent, wherein a color formation reaction of the test reagent in the reagent test region is caused by applying the bodily fluid to the reagent test region.

In a second step 104, the initial numerical analyte result value is compared to an upper threshold value and to a lower threshold value and a final numerical analyte result value is formed either by the unchanged initial numerical analyte result value or by adding or subtracting a bias value to the initial numerical analyte result value depending on an outcome of the comparison.

If the initial numerical analyte result value exceeds the upper threshold value, i.e. is bigger than the upper threshold value, then a preset upper bias is added to the initial numerical analyte result value to form the final numerical analyte result value. Thus, the final numerical analyte result value is increased or enlarged compared to the initial numerical analyte result value. The preset upper bias may be a value that is constant for all initial numerical analyte result values or it may be a value that depends on the initial numerical analyte result value itself and/or the initial numerical analyte result value's distance to the upper threshold value or the like. Exemplary embodiments of the upper bias are described in more detail below.

If the initial numerical analyte result value falls below the lower threshold value, i.e. is smaller than the lower threshold value, then a preset lower bias is subtracted from the initial numerical analyte result value to form the final numerical analyte result value. Thus, the final numerical analyte result value is decreased or made smaller compared to the initial numerical analyte result value. As the upper bias, the preset lower bias may be a value constant for all initial numerical analyte result values or a value depending on another quantity, such as the initial numerical analyte result value itself or its distance to the lower threshold value or the like.

If the initial numerical analyte result value neither exceeds the upper threshold value nor falls below the lower threshold value, i.e. is smaller than the upper threshold value and bigger than the lower threshold value, then the final numerical analyte result value is formed by the unchanged initial numerical analyte result value. Thus, in this case the final numerical analyte result value is identical to the initial numerical analyte result value.

It shall be understood that each threshold value forms part of one of the two ranges separated by the threshold value. For example, in a first embodiment initial numerical analyte result values that are identical to the upper threshold value or to the lower threshold value are kept unchanged. Alternatively, an initial numerical analyte result value being identical to the upper or lower threshold value may be biased.

The method step 104 is followed by a step 106, wherein step 106 proceeds with the final numerical analyte result value resulting from step 104, i.e., a value corresponding to the unchanged initial numerical analyte result value or the biased initial numerical analyte result value.

**In** the steps 106 a measurement result is communicated to the user by displaying the final numerical analyte result value resulting from steps 104 and/or an analyte value range corresponding to the final numerical analyte result value resulting from the steps 104 and/or a message associated with the corresponding analyte value range. An associated message or, in other words, a corresponding message may be any message suitable to inform the user of the determined analyte concentration.

Attributing or determining an analyte value range corresponding to the final numerical analyte result value implies determining an analyte value range that comprises the final numerical analyte result value.

**In** an embodiment, a series of analyte value ranges portioning a measurement range is predefined, i.e., each analyte value range of the series is preset, e.g., by defining range limits portioning the measurement range and separating adjacent analyte value ranges. Again, it shall be understood that each range limit forms part of one of the two analyte value ranges it separates. In a corresponding embodiment, step 106 comprises determining the analyte value range of the series of analyte value ranges that covers, i.e. comprises, the final numerical analyte result value. Step 106 may also comprise determining a message associated with the corresponding analyte value range, e.g. using a look-up-table or the like.

After step 106 the method may be repeated as indicated by arrow 108 determining a new initial numerical analyte result value as well as a new final numerical analyte result value on the bases of the new initial numerical analyte result value.

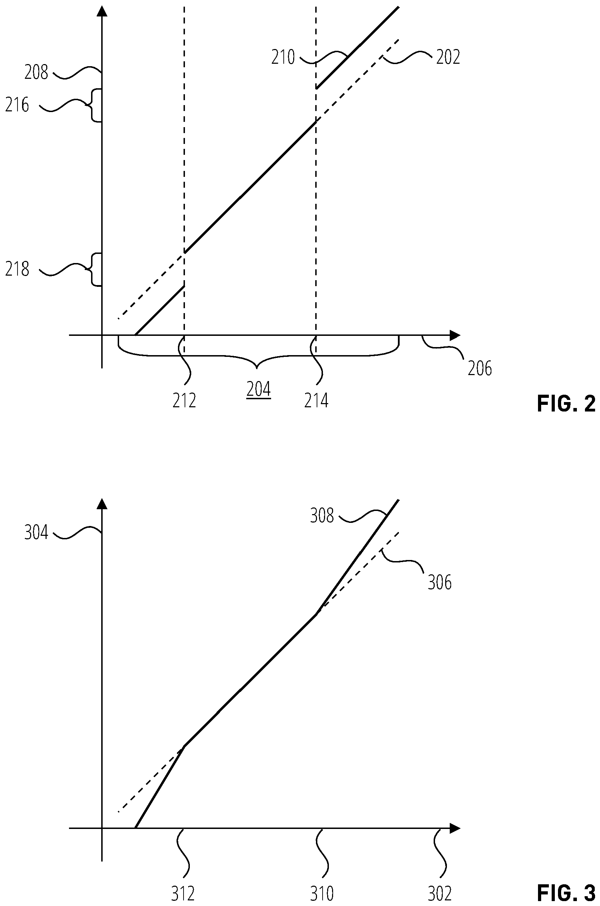

**In** FIG. 2 a first embodiment of the biasing of initial numerical analyte result values to form final numerical analyte result values is illustrated. The dashed line 202 depicts an identity function or identity relation, i.e., the final numerical analyte result value being identical to the unchanged initial numerical analyte result values over the entire measurement range 204. Thus, each initial numerical analyte result value of the measurement range 204 along the abscissa 206 is mapped to an identical final numerical analyte result value on the ordinate 208.

The solid line 210 depicts final numerical analyte result values achieved by biasing by a constant value of initial numerical analyte result values that are smaller than the lower threshold value 212 or bigger than the upper threshold value 214. Correspondingly, the solid line 210 coincides with the dashed line 202 for values bigger than the lower threshold value 212 and smaller than the upper threshold value 214. To form the final numerical analyte result value a constant upper bias 216 is added to all initial numerical analyte result values bigger than the upper threshold value 214 so that the solid line 210 is correspondingly shifted in parallel above the dashed line 202. Furthermore, to form the final numerical analyte result value a constant lower bias 218 is subtracted from all initial numerical analyte result values smaller than the lower threshold value 212 so that the solid line 210 is correspondingly shifted in parallel below the dashed line 202. In the depicted embodiment the upper bias 2016 and the lower bias 218 are identical.

In FIG. 3 a second embodiment of the biasing of initial numerical analyte result values to form final numerical analyte result values is illustrated by depicting the mapping of the initial numerical analyte result values along the abscissa 302 onto final numerical analyte result values along the ordinate 304. The dashed line 306 again depicts an identity function or identity relation as reference. The solid line 308 depicts the biasing of initial numerical analyte result values wherein the size of the bias depends on the initial numerical analyte result value, particularly, on a distance of the initial numerical analyte result value to the respective threshold value.

As depicted in FIG. 3 the upper bias is an upper transition function with function values that are proportional to an absolute value of a difference between the initial numerical analyte result value and the upper threshold values 310. Correspondingly, the lower bias is a lower transition function with function values that are proportional to an absolute value of a difference between the initial numerical analyte result value and the lower threshold value 312.

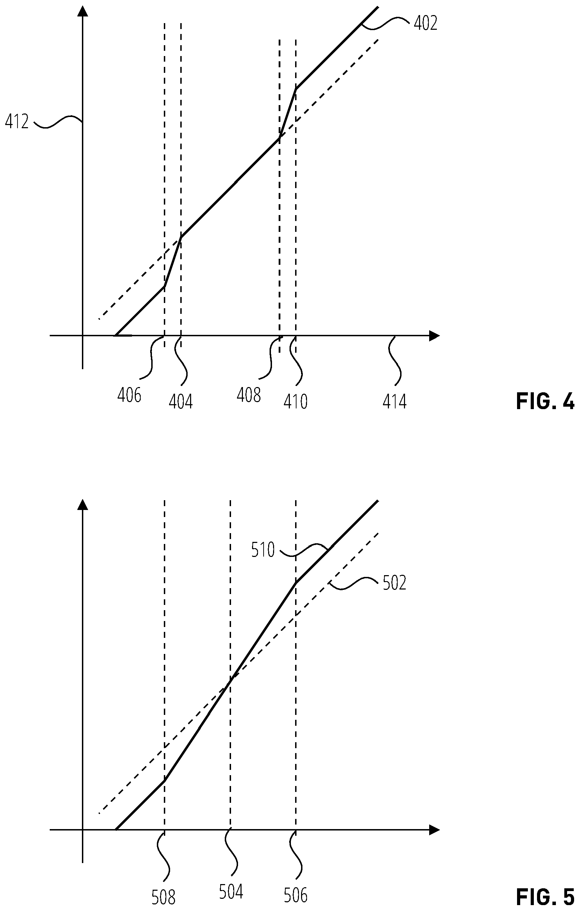

In FIG. 4 a third embodiment of the biasing of initial numerical analyte result values to form final numerical analyte result values is illustrated. As depicted by the solid line 210 the upper bias is an upper transition function and the lower bias is a lower transition function.

The upper transition function and the lower transition function comprise a first region with a gradual increase proportional to an increase of the respective distance of the initial numerical analyte result value to the upper threshold value 408 or lower threshold value 404, respectively, and a second region with a constant value.

In the depicted embodiment the first region extends between the upper threshold value 408 and a greater second upper threshold value 410 or between the lower threshold value 404 and a smaller second lower threshold value 406, respectively, while the second region is adjacent to the first region, covering all values greater than the second upper threshold value 410 or smaller than the second lower threshold value 406, respectively. It shall be understood that each threshold value belongs to one of the two adjacent regions.

In FIG. 5 a fourth embodiment of the biasing of initial numerical analyte result values to form final numerical analyte result value is illustrated, wherein the dashed line 502 depicting the identity function is given as a reference.

In the depicted embodiment the lower threshold value and the upper threshold value are identical, thus coincide with a mutual threshold value 504. The upper bias and the lower bias are an upper transition function and a lower transition function, respectively. Each transition function comprises a first region spanning between the mutual threshold value 504 and a second upper threshold value 506 or a second lower threshold value 508, respectively and a second region covering all initial numerical analyte result values exceeding the second upper threshold value 506 or falling below the second lower threshold value 508, respectively.

In the first region the upper and lower transition function comprise function values that are proportional to a distance of the initial numerical analyte result value to the mutual threshold value 504. In the second region, the function values of the upper and lower transition function have a constant value.

Correspondingly, the solid line 510 depicting the mapping of the initial numerical analyte result values to the final numerical analyte result values comprises a linear progression between the second lower threshold value 508 and the second upper threshold value 506 crossing the dashed line 502 at the mutual threshold value 504. In the regions above the second upper threshold value 506 and below the second lower threshold value 508 the solid line 510 is shifted in parallel above and below the dashed line 502, respectively.

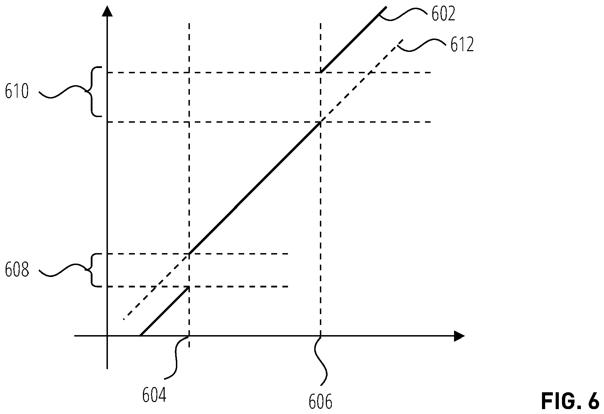

In FIG. 6 a fifth embodiment of the biasing of initial numerical analyte result values to form final numerical analyte result value is illustrated, wherein the dashed line 612 depicting the identity function is given as a reference.

In the depicted embodiment, initial numerical analyte result values smaller than the lower threshold value 604 are decreased by the constant lower bias 608 to form the final numerical analyte result value, as shown by the solid line 602. Initial numerical analyte result values bigger than the upper threshold value 606 are increased by the constant upper bias 610 to form the final numerical analyte result value, wherein the upper bias 610 is bigger than the lower bias 608.

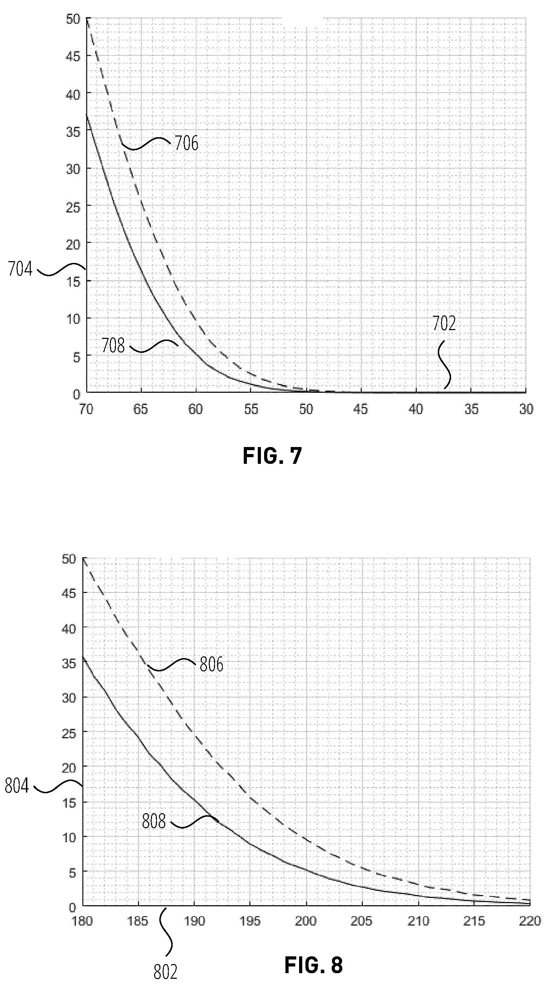

In the graph depicted in FIG. 7 the effect of measurement errors regarding missing critically low analyte concentration states is illustrated using the example of a glucose measurement.

Blood glucose levels below 70 mg/dl may be critical to a person's health and therefore it is particularly important to reduce the risk of falsely determining a non-critical, in range concentration value when the analyte concentration of the actually applied sample of a bodily fluid is out of range, e.g. too low.

In the graph, the abscissa 702 depicts true analyte concentration values of a sample starting from 70 mg/dl and decreasing to 30 mg/dl. The ordinate 704 depicts the probability to determine a concentration value, i.e. a final numerical analyte result value of at least 70 mg/dl or bigger, thus, the probability to miss a critically low analyte concentration state.

Without the inventive biasing, thus taking the initial numerical analyte result values as final numerical analyte result value the probability to actually determine exactly the 70 mg/dl when the applied sample does have an analyte concentration of 70 dl/mg is 50% and gradually decreases for lower actual analyte concentrations as depicted by the dashed line 706. Thus, for actual concentrations below but close to the critical value of 70 mg/dl the probability to miss recognizing and communicating the critical concentration is close to 50%.

The inventive method helps to reduce this probability of missing critical states as illustrated by the solid line 708 in the graph of FIG. 7 by decreasing smaller initial numerical analyte result values to form the final numerical analyte result values.

Corresponding to the graph in FIG. 7 the graph in FIG. 8 illustrates the effect of measurement errors regarding missing critically high analyte concentration states.

In the graph, the abscissa 802 depicts true analyte concentration values of a sample starting from 180 mg/dl and increasing to 220 mg/dl. The ordinate 804 depicts the probability to determine a concentration value, i.e. a final numerical analyte result value, of 180 mg/dl or less, thus, missing a critically high analyte concentration state.

While the dashed line 806 depicts the probability when determining a final numerical analyte result value without biasing any initial numerical analyte result value, the solid line 808 shows that the probability to miss critically high states is reduced by artificially biasing, namely increasing higher initial numerical analyte result values to form final numerical analyte result values.

## Claims

1. A method of determining a final numerical analyte result value corresponding to a concentration of an analyte in a bodily fluid using a mobile device having a processing device, and a display device comprising the steps of:
- determining by the processing device an initial numerical analyte result value from an image of a color formation of a reagent test region (102);
- displaying a final numerical analyte result value and/or an analyte value range of a group of preset analyte value ranges corresponding to the final numerical analyte result value and/or a message corresponding to the corresponding analyte value range (106);
- wherein the final numerical analyte result value is determined (104) by
- comparing the initial numerical analyte result value to at least one upper threshold value and to at least one lower threshold value and
- adding a preset upper bias to the initial numerical analyte result value to form the final numerical analyte result value if the initial numerical analyte result value exceeds the upper threshold value, or
- subtracting a preset lower bias from the initial numerical analyte result value to form the final numerical analyte result value if the initial numerical analyte result value falls below the lower threshold value, or
- using the unchanged initial numerical analyte result value to form the final numerical analyte result value if the initial numerical analyte result value does neither exceed the upper threshold value nor fall below the lower threshold value.

2. The method of claim 1, wherein the upper bias is a constant upper offset value or an upper transition function with function values depending on the initial numerical analyte result value and/or on an absolute value of a difference between the initial numerical analyte result value and the upper threshold value.

3. The method of claim 1 or 2, wherein the lower bias is a constant lower offset value or a lower transition function with function values depending on the initial numerical analyte result value and/or on an absolute value of a difference between the initial numerical analyte result value and the lower threshold value.

4. The method of claim 2 or 3, wherein the lower offset value and the upper offset value are identical or differ from each other.

5. The method of claim 2 or 3, wherein the upper transition function and/or the lower transition function gradually increases with an increase of a distance given by the absolute value of the difference between the numerical analyte result value and the upper threshold value or lower threshold value, respectively.

6. The method of claim 2 or 3, wherein the upper transition function and/or the lower transition function comprises a first region with a gradual increase with an increase of the respective distance and a second region with a constant value.

7. The method of claim 5 or 6, wherein the gradual increase of the upper transition function and/or the lower transition function is proportional to the absolute value of the difference between the numerical analyte result value and the upper threshold value or lower threshold value, respectively.

8. The method of any one of claims 1 to 7, wherein the upper threshold value and the lower threshold value are identical.

9. The method of any one of claims 1 to 8, wherein determining the final numerical analyte result value comprises
- comparing the initial numerical analyte result value to a second upper threshold value and a second lower threshold value and
- using the unchanged initial numerical analyte result value to form the final numerical analyte result value if the initial numerical analyte result value exceeds the second upper threshold value or falls below the second lower threshold value.

10. The method of any one of claims 1 to 9, wherein the analyte is blood glucose and wherein the bodily fluid is either blood or interstitial fluid.

11. A computer program including computer-executable instructions for performing the method according to any one of claims 1 to 10.

12. A non-transitory computer-readable storage medium, the computer-readable storage medium including instructions that when executed by a mobile device, cause the mobile device to perform the method according to any one of claims 1 to 10.

13. A mobile device comprising:
a processing device;
a display device;
a camera; and
a memory storing instructions that, when executed by the processing device, configure the mobile device to perform the method according to any one of claims 1 to 10.

14. A kit for determining a concentration of an analyte in a bodily fluid, the kit comprising at least one mobile device according to claim 13 and at least one optical test strip having at least one test field.

## Patentansprüche

1. Verfahren zum Bestimmen eines numerischen Analytendergebniswertes, der einer Konzentration eines Analyten in einer Körperflüssigkeit entspricht, unter Verwendung einer mobilen Vorrichtung mit einer Verarbeitungsvorrichtung und einer Anzeigevorrichtung, umfassend die folgenden Schritte:
- Bestimmen eines numerischen Analytausgangsergebniswertes aus einem Bild einer Farbbildung einer Reagenztestregion (102) mittels der Verarbeitungsvorrichtung;
- Anzeigen eines numerischen Analytendergebniswertes und/oder eines Analytwertebereiches einer Gruppe von vorgegebenen Analytwertebereichen, die dem numerischen Analytendergebniswert entsprechen, und/oder einer Nachricht, die dem entsprechenden Analytwertebereich (106) entspricht;
- wobei der numerische Analytendergebniswert wie folgt bestimmt wird (104)
- Vergleichen des numerischen Analytausgangsergebniswertes mit mindestens einem oberen Schwellenwert und mit mindestens einem unteren Schwellenwert und
- Addieren eines vorgegebenen oberen Bias zu dem numerischen Analytausgangsergebniswert zum Bilden des numerischen Analytendergebniswertes, wenn der numerische Analytausgangsergebniswert den oberen Schwellenwert überschreitet, oder
- Subtrahieren eines vorgegebenen unteren Bias von dem numerischen Analytausgangsergebniswert zum Bilden des numerischen Analytendergebniswertes, wenn der numerische Analytausgangsergebniswert unter den unteren Schwellenwert fällt, oder
- Verwenden des unveränderten numerischen Analytausgangsergebniswertes zum Bilden des numerischen Analytendergebniswertes, wenn der numerische Analytausgangsergebniswert weder den oberen Schwellenwert überschreitet noch unter den unteren Schwellenwert fällt.

2. Verfahren nach Anspruch 1, wobei das obere Bias ein konstanter oberer Versatzwert oder eine obere Übergangsfunktion mit Funktionswerten, die von dem numerischen Analytausgangsergebniswert und/oder von einem Absolutwert einer Differenz zwischen dem numerischen Analytausgangsergebniswert und dem oberen Schwellenwert abhängen, ist.

3. Verfahren nach Anspruch 1 oder 2, wobei das untere Bias ein konstanter unterer Versatzwert oder eine untere Übergangsfunktion mit Funktionswerten, die von dem numerischen Analytausgangsergebniswert und/oder von einem Absolutwert einer Differenz zwischen dem numerischen Analytausgangsergebniswert und dem unteren Schwellenwert abhängen, ist.

4. Verfahren nach Anspruch 2 oder 3, wobei der untere Versatzwert und der obere Versatzwert identisch oder verschieden voneinander sind.

5. Verfahren nach Anspruch 2 oder 3, wobei die obere Übergangsfunktion und/oder die untere Übergangsfunktion mit einer Zunahme eines Abstands, der von dem Absolutwert der Differenz zwischen dem numerischen Analytergebniswert und dem oberen Schwellenwert bzw. unteren Schwellenwert vorgegeben wird, stufenweise ansteigen.

6. Verfahren nach Anspruch 2 oder 3, wobei die obere Übergangsfunktion und/oder die untere Übergangsfunktion eine erste Region mit einem stufenweisen Anstieg mit einer Zunahme des jeweiligen Abstands und eine zweite Region mit einem konstanten Wert umfassen.

7. Verfahren nach Anspruch 5 oder 6, wobei der stufenweise Anstieg der oberen Übergangsfunktion und/oder der unteren Übergangsfunktion proportional zum Absolutwert der Differenz zwischen dem numerischen Analytergebniswert und dem oberen Schwellenwert bzw. unteren Schwellenwert ist.

8. Verfahren nach einem der Ansprüche 1 bis 7, wobei der obere Schwellenwert und der untere Schwellenwert identisch sind.

9. Verfahren nach einem der Ansprüche 1 bis 8, wobei das Bestimmen des numerischen Analytendergebniswertes Folgendes umfasst
- Vergleichen des numerischen Analytausgangsergebniswertes mit einem zweiten oberen Schwellenwert und einem zweiten unteren Schwellenwert und
- Verwenden des unveränderten numerischen Analytausgangsergebniswertes zum Bilden des numerischen Analytendergebniswertes, wenn der numerische Analytausgangsergebniswert den zweiten oberen Schwellenwert überschreitet oder unter den zweiten unteren Schwellenwert fällt.

10. Verfahren nach einem der Ansprüche 1 bis 9, wobei der Analyt Blutglukose ist und wobei die Körperflüssigkeit entweder Blut oder interstitielle Flüssigkeit ist.

11. Computerprogramm, das von einem Computer ausführbare Anweisungen zum Ausführen des Verfahrens nach einem der Ansprüche 1 bis 10 einschließt.

12. Nichtflüchtiges computerlesbares Speichermedium, wobei das computerlesbare Speichermedium Anweisungen einschließt, die bei der Ausführung von einer mobilen Vorrichtung die mobile Vorrichtung veranlassen, das Verfahren nach einem der Ansprüche 1 bis 10 auszuführen.

13. Mobile Vorrichtung, umfassend:
eine Verarbeitungsvorrichtung;
eine Anzeigevorrichtung;
eine Kamera und
einen Speicher, der Anweisungen speichert, die bei der Ausführung durch die Verarbeitungsvorrichtung die mobile Vorrichtung so konfigurieren, dass sie das Verfahren nach einem der Ansprüche 1 bis 10 ausführt.

14. Kit zum Bestimmen einer Konzentration eines Analyten in einer Körperflüssigkeit, wobei der Kit mindestens eine mobile Vorrichtung nach Anspruch 13 und mindestens einen optischen Teststreifen mit mindestens einem Testfeld umfasst.

## Revendications

1. Procédé de détermination d'une valeur numérique finale du résultat de l'analyte correspondant à une concentration d'un analyte dans un fluide corporel à l'aide d'un dispositif mobile ayant un dispositif de traitement et un dispositif d'affichage comprenant les étapes de :
- détermination par le dispositif de traitement d'une valeur numérique initiale du résultat de l'analyte à partir d'une image d'une formation de couleur d'une région de test de réactif (102) ;
- affichage d'une valeur numérique finale du résultat de l'analyte et/ou d'une plage de valeurs d'analyte d'un groupe de plages de valeurs d'analyte prédéfinies correspondant à la valeur numérique finale du résultat de l'analyte et/ou d'un message correspondant à la plage de valeurs d'analyte (106) correspondante ;
- dans lequel la valeur numérique finale du résultat de l'analyte est déterminée (104) par
- comparaison de la valeur numérique initiale du résultat de l'analyte à au moins une valeur seuil supérieure et à au moins une valeur seuil inférieure et
- ajout d'un biais supérieur prédéfini à la valeur numérique initiale du résultat de l'analyte pour former la valeur numérique finale du résultat de l'analyte si la valeur numérique initiale du résultat de l'analyte dépasse la valeur seuil supérieure, ou
- soustraction d'un biais inférieur prédéfini de la valeur numérique initiale du résultat de l'analyte pour former la valeur numérique finale du résultat de l'analyte si la valeur numérique initiale du résultat de l'analyte tombe en dessous de la valeur seuil inférieure, ou
- utilisation de la valeur numérique initiale inchangée du résultat de l'analyte pour former la valeur numérique finale du résultat de l'analyte si la valeur numérique initiale du résultat de l'analyte ne dépasse pas la valeur seuil supérieure ni ne tombe en dessous de la valeur seuil inférieure.

2. Procédé selon la revendication 1, dans lequel le biais supérieur est une valeur de correction supérieure constante ou une fonction de transition supérieure avec des valeurs de fonction dépendant de la valeur numérique initiale du résultat de l'analyte et/ou d'une valeur absolue d'une différence entre la valeur numérique initiale du résultat de l'analyte et la valeur seuil supérieure.

3. Procédé selon la revendication 1 ou 2, dans lequel le biais inférieur est une valeur de correction inférieure constante ou une fonction de transition inférieure avec des valeurs de fonction dépendant de la valeur numérique initiale du résultat de l'analyte et/ou d'une valeur absolue d'une différence entre la valeur numérique initiale du résultat de l'analyte et la valeur seuil inférieure.

4. Procédé selon la revendication 2 ou 3, dans lequel la valeur de correction inférieure et la valeur de correction supérieure sont identiques ou diffèrent l'une de l'autre.

5. Procédé selon la revendication 2 ou 3, dans lequel la fonction de transition supérieure et/ou la fonction de transition inférieure augmente progressivement avec une augmentation d'une distance donnée par la valeur absolue de la différence entre la valeur numérique du résultat de l'analyte et la valeur seuil supérieure ou la valeur seuil inférieure, respectivement.

6. Procédé selon la revendication 2 ou 3, dans lequel la fonction de transition supérieure et/ou la fonction de transition inférieure comprend une première région avec une augmentation progressive avec une augmentation de la distance respective et une seconde région avec une valeur constante.

7. Procédé selon la revendication 5 ou 6, dans lequel l'augmentation progressive de la fonction de transition supérieure et/ou de la fonction de transition inférieure est proportionnelle à la valeur absolue de la différence entre la valeur numérique du résultat de l'analyte et la valeur seuil supérieure ou la valeur seuil inférieure, respectivement.

8. Procédé selon l'une quelconque des revendications 1 à 7, dans lequel la valeur seuil supérieure et la valeur seuil inférieure sont identiques.

9. Procédé selon l'une quelconque des revendications 1 à 8, dans lequel la détermination de la valeur numérique finale du résultat de l'analyte comprend
- la comparaison de la valeur numérique initiale du résultat de l'analyte à une seconde valeur seuil supérieure et à une seconde valeur seuil inférieure et
- l'utilisation de la valeur numérique initiale inchangée du résultat de l'analyte pour former la valeur numérique finale du résultat de l'analyte si la valeur numérique initiale du résultat de l'analyte dépasse la seconde valeur seuil supérieure ou tombe en dessous de la seconde valeur seuil inférieure.

10. Procédé selon l'une quelconque des revendications 1 à 9, dans lequel l'analyte est le glucose sanguin et dans lequel le fluide corporel est soit du sang soit un fluide interstitiel.

11. Programme informatique comprenant des instructions exécutables par ordinateur permettant de mettre en œuvre le procédé selon l'une quelconque des revendications 1 à 10.

12. Support de stockage lisible par ordinateur non transitoire, le support de stockage lisible par ordinateur comprenant des instructions qui, lorsqu'elles sont exécutées par un dispositif mobile, amènent le dispositif mobile à mettre en œuvre le procédé selon l'une quelconque des revendications 1 à 10.

13. Dispositif mobile comprenant :
un dispositif de traitement ;
un dispositif d'affichage ;
une caméra ; et
une mémoire stockant des instructions qui, lorsqu'elles sont exécutées par le dispositif de traitement, configurent le dispositif mobile pour mettre en œuvre le procédé selon l'une quelconque des revendications 1 à 10.

14. Kit permettant de déterminer une concentration d'un analyte dans un fluide corporel, le kit comprenant au moins un dispositif mobile selon la revendication 13 et au moins une bandelette de test optique ayant au moins un champ de test.
